# EUROPEAN PATENT APPLICATION

(11) **EP 4 243 027 A1**
(43) Date of publication of application: **13.09.2023**
(21) Application number: 23150635.3
(22) Date of filing: 06.01.2023
(51) Int. Cl.: G16C 20/30, G06N 3/00, G16C 20/70

(54) **METHOD AND SYSTEM FOR SELECTING CANDIDATE DRUG COMPOUNDS THROUGH ARTIFICIAL INTELLIGENCE (AI)-BASED DRUG REPURPOSING**

(30) Priority: 10.03.2022 IN 202241013026; 22.04.2022 US 202217660259
(71) Applicant: Wipro Limited, 560 035 Karnataka (IN)
(72) Inventor: MADHUSUDHANAN, Manoj, 560098 Bangalore (IN); CHOYARMADATHIL, Sreekumar, 560103 Bangalore (IN); VG, Kavinila, TAMIL NADU (IN); MADHUSUDHANAN, Rohan, 560098 Bangalore (IN)
(74) Representative: Finnegan Europe LLP

(57) **Abstract**

A system and method for selecting candidate drug compounds for a disorder through Artificial Intelligence (AI)-based drug repurposing is disclosed. The method includes extracting data including target protein-protein interaction complex corresponding to disorder from databases through Natural Language Processing (NLP) algorithm; generating semantic knowledge graph for disorder based on extracted data to identify a set of lead compounds; assigning initial rank to each of set of lead compounds based on historical clinical information and semantic knowledge graph, through predictive model; for each of set of lead compounds, determining binding affinity score through AI-based encoder-decoder model; determining molecular structure stability score based on interaction of molecular structures through deep learning model; and assigning final rank to each of set of lead compounds based on binding affinity score, molecular structure stability score, and intermediate clinical trial data.

## Description

### Technical Field

This disclosure relates generally to drug repurposing, and more particularly to method and system for selecting candidate drug compounds through artificial intelligence (AI)-based drug repurposing.

### BACKGROUND

Conventional drug discovery process is costly and time-consuming and is mostly aimed at designing drugs that selectively target a single molecular entity. However, drugs are known to interact with more than one target sites. A single drug may be used to target multiple proteins and may, therefore, be used to treat new disorders. Drug repurposing is, therefore, a cost and time-efficient method to identify clinically approved drugs to treat new disorders (such as, COVID-19).

Traditionally, drug discovery techniques select a set of candidate drugs for a disorder. The candidate drugs are further tested in various in vitro and in vivo experiments to finally obtain a clinical approval for use. The process generally takes many years to see successful end results. With drug repurposing methods, the candidate drugs identified are pre-approved for human use. Hence, a lot of time is saved in identifying most suitable drugs for treating a disorder.

Conventional techniques fail to predict the correct drug target interaction when crystal structure of the target protein is unavailable. Further, the conventional techniques fail to generate drug sequences based on selected protein targets. Hence, such techniques fail to identify an accurate exploration of lead compounds in a time-efficient manner. Moreover, the conventional techniques deviate from accurate prediction of drug discovery as similarities of sequence of target drug with existing drugs are not identified.

There is, therefore, a need in the present state of art for time and cost-efficient method for identifying candidate drugs for treating disorders.

### SUMMARY OF THE INVENTION

In one embodiment, a method for selecting candidate drug compounds for a disorder through Artificial Intelligence (AI)-based drug repurposing is disclosed. The method includes extracting relevant data corresponding to the disorder from a plurality of databases through an NLP (natural language processing) algorithm. The data includes a target protein-protein interaction complex associated with the disorder. The method further includes generating a semantic knowledge graph for the disorder based on the extracted data to identify a set of lead compounds corresponding to the target protein-protein interaction complex. The method further includes assigning an initial rank to each of the set of lead compounds based on historical clinical information of each of the set of lead compounds and the semantic knowledge graph, through a predictive model. The predictive model includes at least one of a clustering algorithm and a probabilistic algorithm. The method further includes calculating a binding affinity score corresponding to each of the set of lead compounds and the target protein-protein interaction complex through an AI-based encoder-decoder model. For each of the set of lead compounds, the method further includes determining a molecular structure stability score based on interaction of a molecular structure of a lead compound with a molecular structure of the target protein-protein interaction complex through a deep learning model. The method further includes assigning a final rank to each of the set of lead compounds based on the binding affinity score, the molecular structure stability score, and intermediate clinical trial data corresponding to each of the set of lead compounds.

In one embodiment, a system for selecting candidate drug compounds for a disorder through AI-based drug repurposing is disclosed. In one example, the system may include a processor and a computer-readable medium communicatively coupled to the processor. The computer-readable medium may store processor-executable instructions, which, on execution, cause the processor to extract relevant data corresponding to the disorder from a plurality of databases through an NLP algorithm. The data includes a target protein-protein interaction complex associated with the disorder. The processor-executable instructions, on execution, further cause the processor to generate a semantic knowledge graph for the disorder based on the extracted data to identify a set of lead compounds corresponding to the target protein-protein interaction complex. The processor-executable instructions, on execution, further cause the processor to assign an initial rank to each of the set of lead compounds based on historical clinical information of each of the set of lead compounds and the semantic knowledge graph, through a predictive model. The predictive model includes at least one of a clustering algorithm and a probabilistic algorithm. The processor-executable instructions, on execution, further cause the processor to calculate a binding affinity score corresponding to each of the set of lead compounds and the target protein-protein interaction complex through an AI-based encoder-decoder model. For each of the set of lead compounds, the processor-executable instructions, on execution, further cause the processor to determine a molecular structure stability score based on interaction of a molecular structure of a lead compound with a molecular structure of the target protein-protein interaction complex through a deep learning model. The processor-executable instructions, on execution, further cause the processor to assign a final rank to each of the set of lead compounds based on the binding affinity score, the molecular structure stability score, and intermediate clinical trial data corresponding to each of the set of lead compounds.

In one embodiment, a non-transitory computer-readable medium storing computer-executable instructions for selecting candidate drug compounds for a disorder through AI-based drug repurposing is disclosed. The stored instructions, when executed by a processor, cause the processor to perform operations including extracting relevant data corresponding to the disorder from a plurality of databases through an NLP algorithm. The data includes a target protein-protein interaction complex associated with the disorder. The operations further include generating a semantic knowledge graph for the disorder based on the extracted data to identify a set of lead compounds corresponding to the target protein-protein interaction complex. The operations further include assigning an initial rank to each of the set of lead compounds based on historical clinical information of each of the set of lead compounds and the semantic knowledge graph, through a predictive model. The predictive model includes at least one of a clustering algorithm and a probabilistic algorithm. The operations further include calculating a binding affinity score corresponding to each of the set of lead compounds and the target protein-protein interaction complex through an AI-based encoder-decoder model. For each of the set of lead compounds, the operations further include determining a molecular structure stability score based on interaction of a molecular structure of a lead compound with a molecular structure of the target protein-protein interaction complex through a deep learning model. The operations further include assigning a final rank to each of the set of lead compounds based on the binding affinity score, the molecular structure stability score, and intermediate clinical trial data corresponding to each of the set of lead compounds.

### BRIEF DESCRIPTION OF THE DRAWINGS

The accompanying drawings, which are incorporated in and constitute a part of this disclosure, illustrate exemplary embodiments and, together with the description, serve to explain the disclosed principles.
**FIG. 1** is a block diagram of an exemplary system for selecting candidate drug compounds for a disorder through Artificial Intelligence (AI)-based drug repurposing, in accordance with some embodiments of the present disclosure.
**FIG. 2** is a functional block diagram of an exemplary system for selecting candidate drug compounds for a disorder through AI-based drug repurposing, in accordance with some embodiments of the present disclosure.
**FIG. 3** illustrates a flow diagram of an exemplary method for selecting candidate drug compounds for a disorder through AI-based drug repurposing, in accordance with some embodiments of the present disclosure.
**FIG. 4** illustrates an exemplary control logic for assigning an initial rank to each of a set of lead compounds through a deep learning algorithm, in accordance with an embodiment of the present disclosure.
**FIG. 5** illustrates a flow diagram of an exemplary method for calculating a binding affinity score through an AI-based encoder-decoder model, in accordance with some embodiments of the present disclosure.
**FIG. 6** illustrates an exemplary AI-based encoder-decoder model for calculating a binding affinity score, in accordance with an embodiment of the present disclosure.
**FIG. 7** illustrates a flow diagram of an exemplary method for determining a molecular structure stability score of each of a set of lead compounds through a deep learning model, in accordance with some embodiments of the present disclosure.
**FIG. 8** illustrates a deep learning model for determining a molecular structure stability score of each of a set of lead compounds, in accordance with an embodiment of the present disclosure.
**FIG. 9** illustrates a flow diagram of an exemplary method for assigning a final rank to each of a set of lead compounds, in accordance with an embodiment of the present disclosure.
**FIG. 10** illustrates a flow diagram of a detailed exemplary method for selecting candidate drug compounds for a disorder through AI-based drug repurposing, in accordance with an embodiment of the present disclosure.
**FIG. 11** is a block diagram of an exemplary computer system for implementing embodiments consistent with the present disclosure.

### DETAILED DESCRIPTION

Exemplary embodiments are described with reference to the accompanying drawings. Wherever convenient, the same reference numbers are used throughout the drawings to refer to the same or like parts. While examples and features of disclosed principles are described herein, modifications, adaptations, and other implementations are possible without departing from the and scope of the disclosed embodiments. It is intended that the following detailed description be considered as exemplary only, with the true scope being indicated by the following claims. Additional illustrative embodiments are listed.

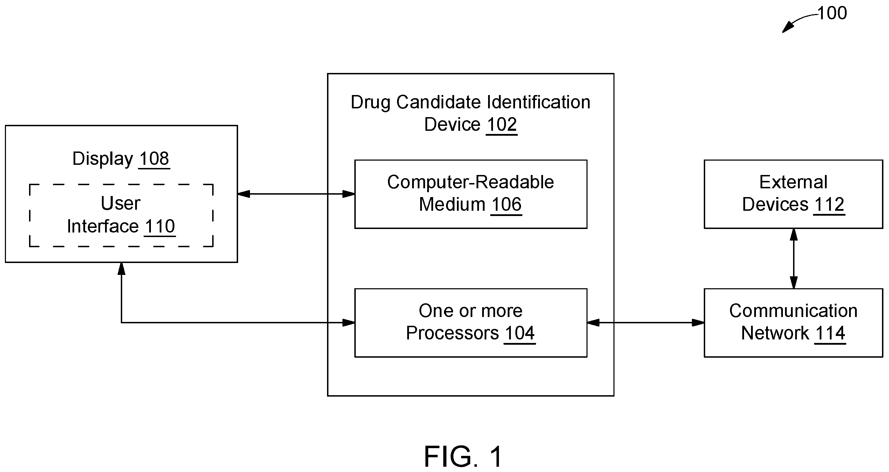

Referring now to FIG. 1, an exemplary system 100 for selecting candidate drug compounds for a disorder through Artificial Intelligence (AI)-based drug repurposing is illustrated, in accordance with some embodiments. The system 100 may implement a drug candidate identification device 102 (for example, server, desktop, laptop, notebook, netbook, tablet, smartphone, mobile phone, or any other computing device), in accordance with some embodiments of the present disclosure. The drug candidate identification device 102 may select candidate drug compounds for a disorder through AI-based drug repurposing using protein-protein interaction analysis and molecular structure stability analysis.

In some embodiments, the drug candidate identification device 102 may include one or more processors 104 and a computer-readable medium 106 (for example, a memory). The computer-readable medium 106 may include one or more databases (not shown). Further, the computer-readable medium 106 may store instructions that, when executed by the one or more processors 104, cause the one or more processors 104 to select candidate drug compounds for a disorder through AI-based drug repurposing, in accordance with aspects of the present disclosure. The computer-readable medium 106 may also store various data (for example, disease data, predictive model data, AI-based encoder-decoder model data, molecular structure data, intermediate clinical trial data, and the like) that may be captured, processed, and/or required by the system 100.

The system 100 may further include a display 108. The system 100 may interact with a user via a user interface 110 accessible via the display 108. The system 100 may also include one or more external devices 112. In some embodiments, the drug candidate identification device 102 may interact with the one or more external devices 112 over a communication network 114 for sending or receiving various data. The external devices 112 may include, but may not be limited to, a remote server, a digital device, or another computing system.

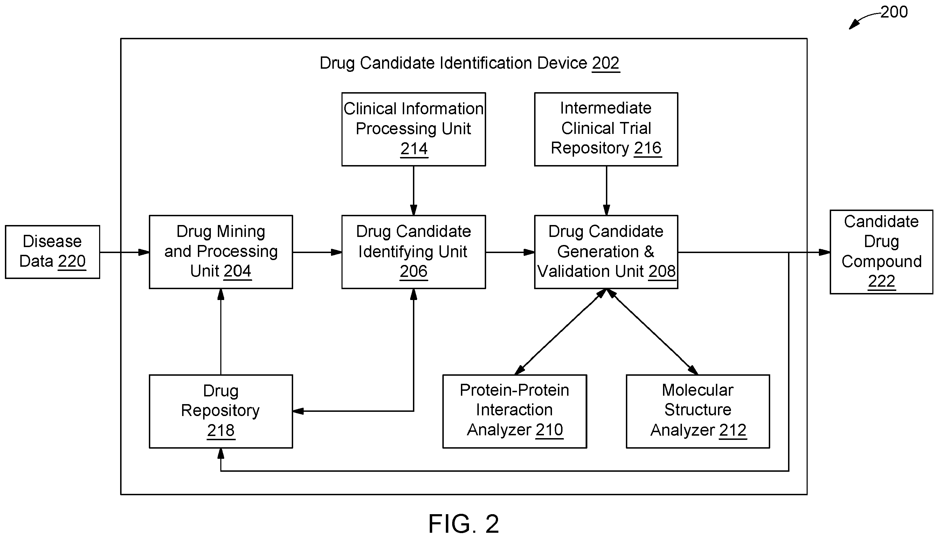

Referring now to FIG. 2, a functional block diagram of an exemplary system 200 for selecting candidate drug compounds for a disorder through AI-based drug repurposing is illustrated, in accordance with some embodiments of the present disclosure. The system 200 includes a drug candidate identification device 202. It may be noted that the drug candidate identification device 202 is analogous to the drug candidate identification device 102 of the system 100. The drug candidate identification device 202 includes a drug mining and processing unit 204, a drug candidate identifying unit 206, a drug candidate generation and validation unit 208, a protein-protein interaction analyzer 210, a molecular structure analyzer 212, a clinical information processing unit 214, an intermediate clinical trial repository 216, and a drug repository 218.

The drug mining and processing unit 204 extracts disease data 220 including valid and relevant information corresponding to a target disorder from standard data sources or through user input. By way of an example, the disease data 220 includes a target protein-protein interaction complex associated with the target disorder. The drug mining and processing unit 204 implements an NLP algorithm to explore larger resources and extract valid and relevant information about the target disorder, drug details from databases such as, but not limited to, PubMed, DrugBank, PharmGKB, and the like.

Upon collecting the disease data 220, the drug mining and processing unit 204 identifies a set of lead compounds, corresponding diseases, and target proteins using a custom trained Bidirectional Encoder Representations from Transformers (BERT) model built from Bio-BERT embeddings as a Named Entity Recognizer (NER). After NER model, the drug mining and processing unit 204 uses distributional semantics (such as, pharmacogenomic relationships) to construct more complete lexicons of drugs, genes, and phenotypes. Further, the drug mining and processing unit 204 uses the constructed lexicons in identifying drug-gene, gene-gene, and gene-phenotype relationships. In an embodiment, the drug mining and processing unit 204 may receive data related to drug-gene, gene-gene, and gene-phenotype relationships.

Further, the drug mining and processing unit 204 plots an extensive semantic knowledge graph from the drug-gene, gene-gene, and gene-phenotype relationships. In an embodiment, the drug mining and processing unit 204 uses Concordance Index (CI) score as a metric for validating drug-gene, gene-gene, and gene-phenotype relationships and plots an extensive semantic knowledge graph based on the validated relationships. Further, the drug mining and processing unit 204 identifies valid enzymes and proteins from the semantic knowledge graph. Further, the drug mining and processing unit 204 validates the identified proteins based on human-curated data from PharmGKB. Further, the drug mining and processing unit 204 matches the identified proteins with drugs from the drug repository 218 and selects a set of lead compounds to limit searching scope. Each of the set of lead compounds is a matching drug with respect to one or more of the identified proteins. Further, the drug mining and processing unit 204 sends the set of lead compounds to the drug candidate identifying unit 206.

The clinical information processing unit 214 processes and stores clinical properties of the set of lead compounds (such as, stage of administration, route of administration, oral bio-availability, half-life, mechanism of action, renal excretion, adverse effects, toxicity, comorbid safety, physical properties, etc.) from Drug Bank. In an embodiment, the clinical information processing unit 214 stores and processes each of the set of lead compounds and associated pharmacokinetic and pharmacodynamic properties.

The drug candidate identifying unit 206 receives the set of lead compounds from the drug mining and processing unit 204. Further, the drug candidate identifying unit 206 creates Gaussian Mixture Models (GMMs) based on associated pharmacokinetic and pharmacodynamic properties stored in the clinical information processing unit 214 to classify each of the set of lead compounds into one or more clusters. As will be appreciated, a GMM is based on an unsupervised clustering algorithm. Further, the drug candidate identifying unit 206 assigns a custom score to each of the one or more clusters based on available historical clinical feature information and validates each of the one or more clusters based on historical information of other existing diseases.

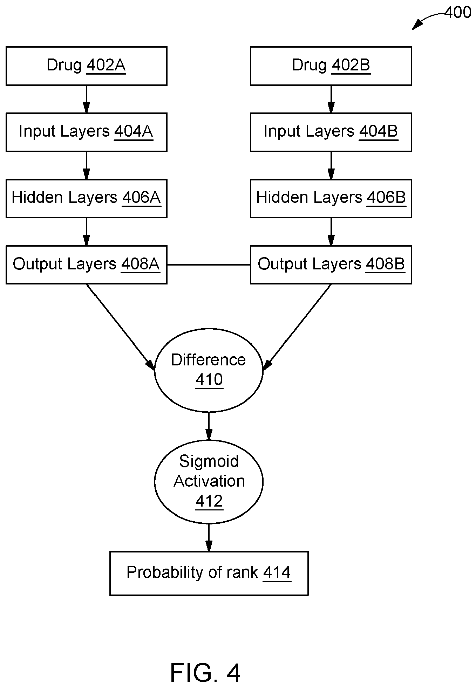

Upon assigning the custom score, the drug candidate identifying unit 206 applies a combination of deep learning-based ranking algorithms to assign an initial rank to each of the set of lead compounds corresponding to the target disorder. In an embodiment, the combination of deep learning-based ranking algorithms includes RankNet and LambdaMart. This is further explained in conjunction with FIG. 4.

The drug candidate generation and validation unit 208 receives the set of lead compounds from the drug candidate identifying unit 206. The drug candidate generation and validation unit 208 sends each of the set of lead compounds to the protein-protein interaction analyzer 210 and receives a corresponding binding affinity score of a lead compound with the target protein-protein interaction complex. Further, the drug candidate generation and validation unit 208 sends the binding affinity score of each of the set of lead compounds to a predefined ranking model with a higher weightage to adjust the initial rank of the set of lead compounds with respect to the disorder. Therefore, importance of drug-target interactions is considered in the predefined ranking model.

The protein-protein interaction analyzer 210 analyzes protein-protein interaction of a lead compound with the target protein-protein interaction complex by predicting the binding affinity score of the lead compound with amino acid sequence corresponding to the target protein-protein interaction complex.

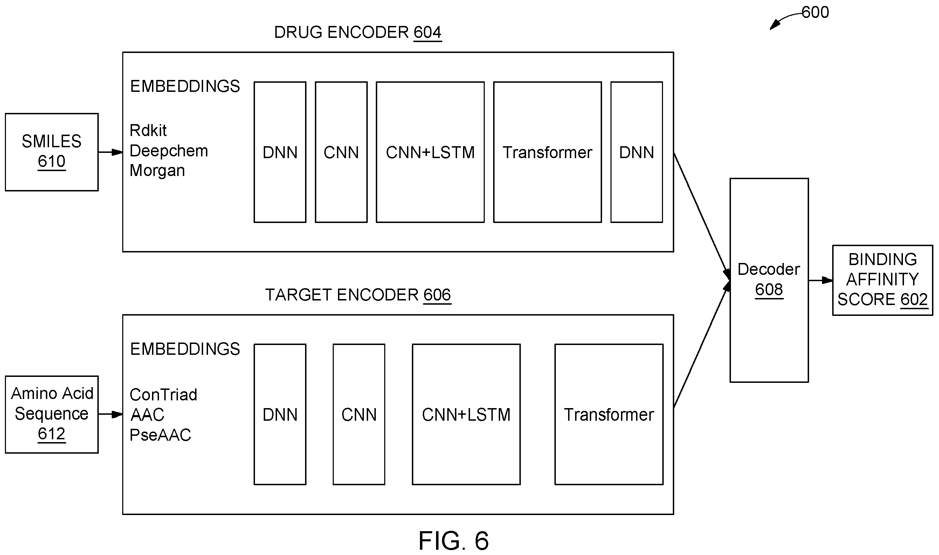

The protein-protein interaction analyzer 210 generates drug and protein embeddings through AI-based encoder networks and concatenates the drug and protein embeddings into a decoder network to predict the binding affinity score of each of the set of lead compounds. This is further explained in detail in conjunction with FIG. 6.

Further, the drug candidate generation and validation unit 208 sends each of the set of lead compounds to the molecular structure analyzer 212 and receives a corresponding molecular structure stability score. The molecular structure stability score indicates compatibility of a lead compound with the target protein-protein interaction complex.

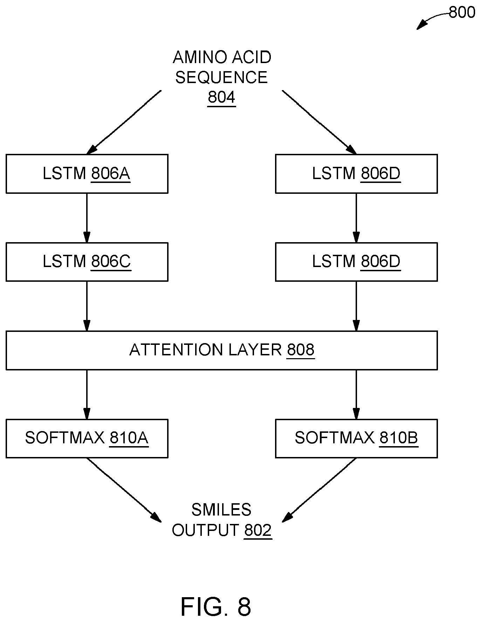

The molecular structure analyzer 212 assesses molecular structure stability of each of the set of lead compounds to improve ranking of the set of lead compounds. The molecular structure analyzer 212 generates a novel compound which ideally targets the target proteins using deep learning algorithms. Further, the molecular structure analyzer 212 assesses molecular structure stability of the novel compound by comparing the novel compound with existing drug compounds. This is further explained in detail in conjunction with FIG. 8.

Upon obtaining a structurally compatible novel compound in Simplified Molecular Input Line Entry System (SMILES) format from the molecular structure analyzer 212, the drug candidate generation and validation unit 208 uses previously available drug encoders to estimate similarities of the novel compound with the set of lead compounds. Based on the estimated similarities, the drug candidate generation and validation unit 208 assigns cosine similarity scores to each of the set of lead compounds.

Further, the drug candidate generation and validation unit 208 uses input from each of the protein-protein interaction analyzer 210 and the molecular structure analyzer 212 as a feature in ranking algorithm to adjust the initial rank of each of the set of lead compounds for identifying valid set of lead compounds with respect to the target disorder.

Further, the drug candidate generation and validation unit 208 receives intermediate clinical trial data corresponding to each of the set of lead compounds from the intermediate clinical trial repository 216. The intermediate clinical trial data includes clinical trial data for a lead compound when used to treat the target disorder. Further, the drug candidate generation and validation unit 208 assigns a final rank to each of the set of lead compounds based on the associated binding affinity score, the molecular structure stability score, and the intermediate clinical trial data. The drug candidate generation and validation unit 208 outputs the final-ranked set of lead compounds and the corresponding intermediate clinical trial data. Additionally, the drug candidate generation and validation unit 208 updates the drug repository 218 with the corresponding intermediate clinical trial data. Based on the final rank, a candidate drug compound 222 corresponding to the target disorder may be identified from the set of lead compounds.

It should be noted that all such aforementioned modules 204 - 218 may be represented as a single module or a combination of different modules. Further, as will be appreciated by those skilled in the art, each of the modules 204 - 218 may reside, in whole or in parts, on one device or multiple devices in communication with each other. In some embodiments, each of the modules 204 - 218 may be implemented as dedicated hardware circuit comprising custom application-specific integrated circuit (ASIC) or gate arrays, off-the-shelf semiconductors such as logic chips, transistors, or other discrete components. Each of the modules 204 - 218 may also be implemented in a programmable hardware device such as a field programmable gate array (FPGA), programmable array logic, programmable logic device, and so forth. Alternatively, each of the modules 204 - 218 may be implemented in software for execution by various types of processors (e.g., one or more processors 104). An identified module of executable code may, for instance, include one or more physical or logical blocks of computer instructions, which may, for instance, be organized as an object, procedure, function, or other construct. Nevertheless, the executables of an identified module or component need not be physically located together, but may include disparate instructions stored in different locations which, when joined logically together, include the module and achieve the stated purpose of the module. Indeed, a module of executable code could be a single instruction, or many instructions, and may even be distributed over several different code segments, among different applications, and across several memory devices.

As will be appreciated by one skilled in the art, a variety of processes may be employed for selecting candidate drug compounds for a disorder through AI-based drug repurposing. For example, the exemplary system 100 and the associated drug candidate identification device 102 may select candidate drug compounds for a disorder through AI-based drug repurposing by the processes discussed herein. In particular, as will be appreciated by those of ordinary skill in the art, control logic and/or automated routines for performing the techniques and steps described herein may be implemented by the system 100 and the drug candidate identification device 102 either by hardware, software, or combinations of hardware and software. For example, suitable code may be accessed and executed by the one or more processors on the system 100 to perform some or all of the techniques described herein. Similarly, application specific integrated circuits (ASICs) configured to perform some or all of the processes described herein may be included in the one or more processors 104 on the system 100.

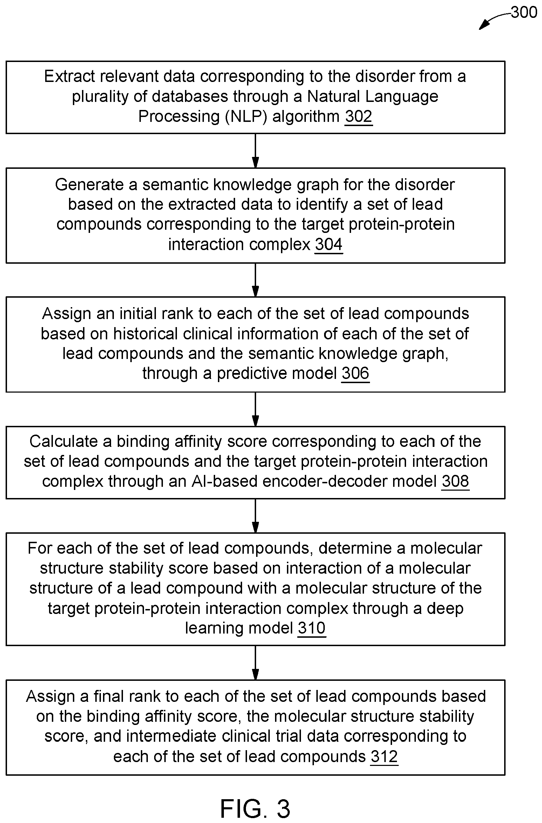

Referring now to FIG. 3, an exemplary method 300 for selecting candidate drug compounds for a disorder through AI-based drug repurposing is depicted via flowchart, in accordance with some embodiments of the present disclosure. In an embodiment, the method 300 may be implemented by the drug candidate identification device 102. The method 300 includes extracting relevant data corresponding to the disorder from a plurality of databases through an NLP algorithm, at step 302. The data includes a target protein-protein interaction complex associated with the disorder. Further, the method 300 includes generating a semantic knowledge graph for the disorder based on the extracted data to identify a set of lead compounds corresponding to the target protein-protein interaction complex, at step 304.

For generating the semantic knowledge graph for the disorder, the method 300 includes, but not limited to, steps of text extraction, tokenization, entity extraction, semantics, and knowledge graph generation. To identify a set of lead compounds corresponding to the target protein-protein interaction complex, the method 300 includes determining one or more target proteins from the target protein-protein interaction complex. Further, the method 300 includes validating the one or more target proteins based on manually curated databases. Further, upon successfully validating, the method 300 includes identifying the set of lead compounds corresponding to each of the one or more target proteins based on drug repositories.

Further, the method 300 includes assigning an initial rank to each of the set of lead compounds based on historical clinical information of each of the set of lead compounds and the semantic knowledge graph, through a predictive model, at step 306. The predictive model includes at least one of a clustering algorithm and a probabilistic algorithm.

For assigning an initial rank to each of the set of lead compounds, the method 300 includes extracting pharmacokinetic and pharmacodynamic properties corresponding each of the set of lead compounds from the semantic knowledge graph. Further, the method 300 includes classifying each of the set of lead compounds into one or more clusters based on the pharmacokinetic and pharmacodynamic properties through a clustering algorithm. Further, the method 300 includes assigning a custom score to each of the one or more clusters based on the historical clinical information of each of the set of lead compounds. Further, the method 300 includes assigning the initial rank to each of the set of lead compounds in each of the one or more clusters through a probabilistic model.

Further, the method 300 includes calculating a binding affinity score corresponding to each of the set of lead compounds and the target protein-protein interaction complex through an AI-based encoder-decoder model, at step 308. Further, for each of the set of lead compounds, the method 300 includes determining a molecular structure stability score based on interaction of a molecular structure of a lead compound with a molecular structure of the target protein-protein interaction complex through a deep learning model, at step 310.

Further, the method 300 includes assigning a final rank to each of the set of lead compounds based on the binding affinity score, the molecular structure stability score, and intermediate clinical trial data corresponding to each of the set of lead compounds, at step 312.

Referring now to FIG. 4, an exemplary control logic 400 for assigning an initial rank to each of a set of lead compounds (for example, a drug 402A and a drug 402B) through a deep learning algorithm is depicted via a flow chart, in accordance with an embodiment of the present disclosure. In an embodiment, the control logic 400 may be implemented by the drug candidate identification device 102 or the drug candidate identification device 202.

The drug candidate identifying unit 206 of the drug candidate identification device 202 classifies each of the set of lead compounds into one or more clusters based on the associated pharmacokinetic and pharmacodynamic properties (obtained from the semantic knowledge graph). Further, the drug candidate identifying unit 206 assigns an initial rank to each of the set of lead compounds corresponding to the target disorder through a combination of deep learning-based ranking algorithms (such as, RankNet and LambdaMart).

For example, a cluster includes the drug 402A and the drug 402B. The combination of deep learning-based algorithms determines the initial rank for each of lead compounds within a cluster. In an embodiment, the combination of deep learning-based algorithms assigns the initial rank through a pairwise regression-based model. The pairwise regression-based model includes two neural networks, first neural network for the drug 402A and second neural network for the drug 402B. In some exemplary scenarios, a cluster may include more than two lead compounds. In such scenarios, the combination of deep learning-based algorithms may assign the initial rank to the lead compounds based on analysis of the lead compounds in pairs.

Each neural network includes input layers (for example, input layers 404A corresponding to the drug 402A and input layers 404B corresponding to the drug 402B), hidden layers (for example, hidden layers 406A corresponding to the drug 402A and hidden layers 406B corresponding to the drug 402B), and output layers (for example, output layers 408A corresponding to the drug 402A and output layers 408B corresponding to the drug 402B).

The control logic 400 includes receiving the drug 402A and the drug 402B by the input layers 404A and the input layers 404B, respectively. Further, the control logic 400 includes comparing the drug 402A with the drug 402B by the output layers 408A and the output layers 408B based on the associated pharmacokinetic and pharmacodynamic properties.

Further, the control logic 400 includes determining a difference 410 between the drug 402A and the drug 402B based on the comparing. Further, the control logic 400 includes sending the difference 410 to a sigmoid activation 412. Further, the control logic 400 includes determining a probability of rank 414 for the drug 402A and the drug 402B through the sigmoid activation 412. In an embodiment, the probability of rank 414 indicates probability that the initial rank of drug 402A is higher than the initial rank of the drug 402B.

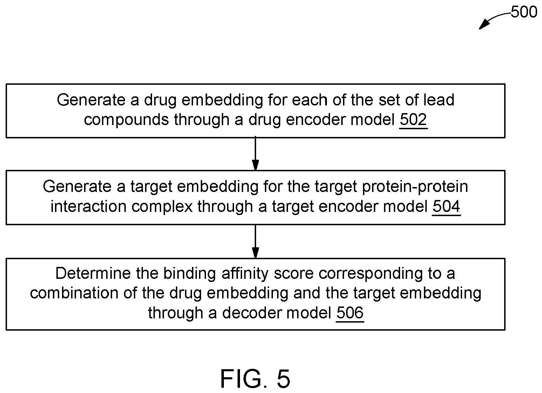

Referring now to FIG. 5, an exemplary method 500 for calculating a binding affinity score through an AI-based encoder-decoder model is depicted via a flow chart, in accordance with some embodiments of the present disclosure. In an embodiment, the method 500 may be implemented by the drug candidate identification device 102. In an embodiment, the method 500 includes identifying protein-ligand interactions of viral protein and host protein from different combinations by estimating the binding affinity score. Further, the method 500 includes generating drug embeddings for each of the set of lead compounds through a drug encoder model, at step 502. Further, the method 500 includes generating target embeddings for the target protein-protein interaction complex through a target encoder model, at step 504. Further, the method 500 includes determining the binding affinity score corresponding to a combination of the drug embedding and the target embedding through a decoder model, at step 506.

Referring now to FIG. 6, an exemplary AI-based encoder-decoder model 600 for calculating a binding affinity score 602 is illustrated, in accordance with an embodiment of the present disclosure. The AI-based encoder-decoder model 600 includes a drug encoder 604, a target encoder 606, and a decoder 608.

The AI-based encoder model 600 identifies protein-ligand interactions of viral protein and host protein from different combinations by estimating the binding affinity score 602 using a deep learning-based approach. Usually, the binding affinity score 602 is determined experimentally and using 3D structural simulations on AutoDock Vina and SurFlex Dock. Further, such 3D simulations are used with chalcogen and halogen bondings for validation of the binding affinity score 602 on AutoDock Vina. A dataset (such as, PDBbind dataset obtained from PDBbind database which is a collection of experimentally measured binding affinity scores for the available biomolecular complexes) may be used to estimate new protein-ligand interactions. Further, the protein-ligand complex may be retrieved as a .pdb file and subsequently, a .pdbqt file (which includes partial charges and atom types). The dataset includes important binding analyzer features (such as, electrostatic interactions, hydrogen bonds, binding pocket flexibility, salt bridges, pie interactions, rotatable bonds, distance between them (restricting to 2.5 to 4 Angstorms), etc.).

The drug encoder 604 receives SMILES 610 string of a lead compound. Further, the drug encoder 604 generates drug embeddings. The drug encoder 604 includes classical cheminformatics fingerprints, such as, RDKit 2D, Deepchem, Morgan, and the like, with a Deep Neural Network (DNN) on top of the cheminformatics fingerprints, and 1-dimensional Convolutional Neural Network (CNN) on the SMILES 610 string, CNN with Long Short-Term Memory (LSTM) to leverage the sequential order, a transformer encoder for sub-structure partition, and a DNN to address to any molecular graph from the SMILES string.

The target encoder 606 receives amino acid sequence 612 of protein-ligand complex. The target encoder 606 generates protein embeddings. The target encoder 606 includes DNN on classical computational biology fingerprints, such as, Conjoint Triad, AAC, Pse AAC, CNN, and the like on the amino acid sequence 612, LSTM on top of CNN, and a transformer for sub-sequence fingerprint.

Further, the drug encoder 604 and target encoder 606 send the drug embeddings and protein embeddings, respectively, to the decoder 608. The decoder 608 concatenates the drug embeddings and the protein embeddings to predict the binding affinity score 602. These two encoder outputs are concatenated into a decoder network to obtain a binding affinity score 602. Root Mean Square Error (RMSE) is loss function of entire architecture and CI score may be used to validate the predicted interactions.

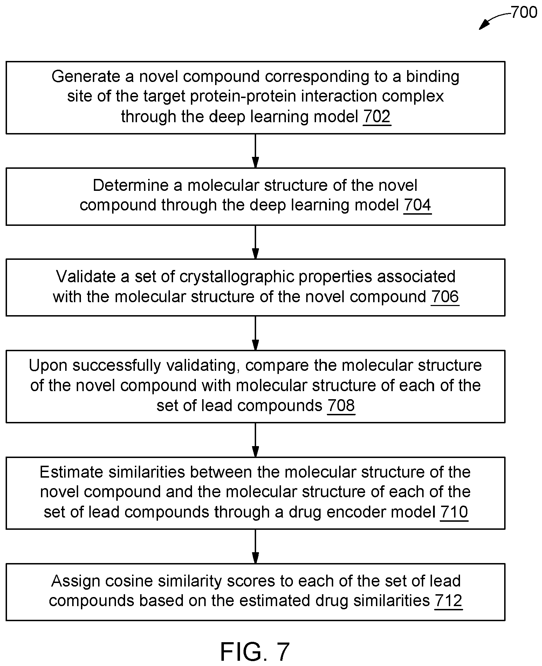

Referring now to FIG. 7, an exemplary method 700 for determining a molecular structure stability score of each of a set of lead compounds through a deep learning model is depicted via a flow chart, in accordance with some embodiments of the present disclosure. In an embodiment, the method 700 may be implemented by the drug candidate identification device 102. The method 700 includes generating a novel compound corresponding to a binding site of the target protein-protein interaction complex through the deep learning model, at step 702. The binding affinity score of the novel compound with the target protein-protein interaction complex is above a predefined threshold.

Further, the method 700 includes determining a molecular structure of the novel compound through the deep learning model, at step 704. Further, the method 700 includes validating a set of crystallographic properties associated with the molecular structure of the novel compound, at step 706. Further, upon successfully validating, the method 700 includes comparing the molecular structure of the novel compound with molecular structure of each of the set of lead compounds, at step 708.

Further, the method 700 includes estimating similarities between the molecular structure of the novel compound and the molecular structure of each of the set of lead compounds through a drug encoder model, at step 710. Further, the method 700 includes assigning cosine similarity scores to each of the set of lead compounds based on the estimated drug similarities, at step 712.

Referring now to FIG. 8, an exemplary deep learning model 800 for determining a molecular structure stability score of each of a set of lead compounds is illustrated, in accordance with an embodiment of the present disclosure. The deep learning model 800 generates a SMILES output 802 of a novel compound corresponding to an amino acid sequence 804 of a protein-ligand complex. The deep learning model 800 includes one more layers of LSTM (such as, LSTM 806A, LSTM 806B, LSTM 806C, and LSTM 806D), an attention layer 808, and one or more layers of SoftMax (such as, SoftMax 810A and SoftMax 810B).

As will be appreciated, LSTM with attention is more efficient to estimate the SMILES output 802 of the novel compound since input data includes amino acid sequence 804 of the protein-ligand complex.

Upon generating the novel compound corresponding to the target protein-protein interaction complex, the molecular structure analyzer 212 generates a molecular structure for the novel compound using a similar attention model. Further, the molecular structure analyzer 212 validates crystallographic properties of the molecular structure. Training data is used to verify the crystallographic properties. The molecular structure analyzer 212 collects common physiochemical features and applies Principal Component Analysis (PCA) on the data to determine whether the molecular structure of the novel compound is transformed accordingly.

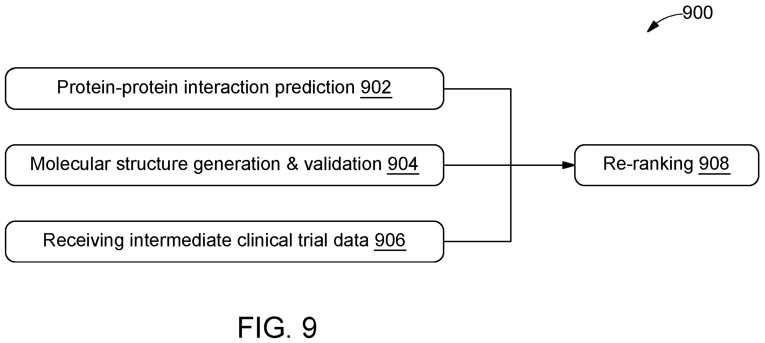

Referring now to FIG. 9, an exemplary method 900 for assigning a final rank to each of a set of lead compounds is depicted via a flow chart, in accordance with an embodiment of the present disclosure. In an embodiment, the method 900 may be implemented by the drug candidate identification device 102.

The method 900 includes protein-protein interaction prediction, at step 902. The protein-protein interaction analyzer 210 determines a binding affinity score for each of the set of lead compounds corresponding to the target protein-protein interaction complex. Further, the protein-protein interaction analyzer 210 sends the binding affinity score to the drug candidate generation and validation unit 208.

Further, the method 900 includes molecular structure generation and validation, at step 904. The molecular structure analyzer 212 determines a molecular structure stability score for each of the set of lead compounds corresponding to the target protein-protein interaction complex. Further, the molecular structure analyzer 212 sends the molecular structure stability score to the drug candidate generation and validation unit 208.

Further, the method 900 includes receiving intermediate clinical trial data, at step 906. The drug candidate generation and validation unit 208 receives intermediate clinical trial data from the intermediate clinical trial repository 216. It may be noted that the steps 902 - 906 may be performed in parallel or sequentially.

Further, the method 900 includes re-ranking, at step 908. The drug candidate generation and validation unit 208 assigns a final rank to each of the set of lead compounds based on the associated binding affinity score, the molecular structure stability score, and the intermediate clinical trial data. Based on final rank assigned to each of a set of lead compounds, the method 900 includes identifying a candidate drug compound 222 corresponding to the target disorder.

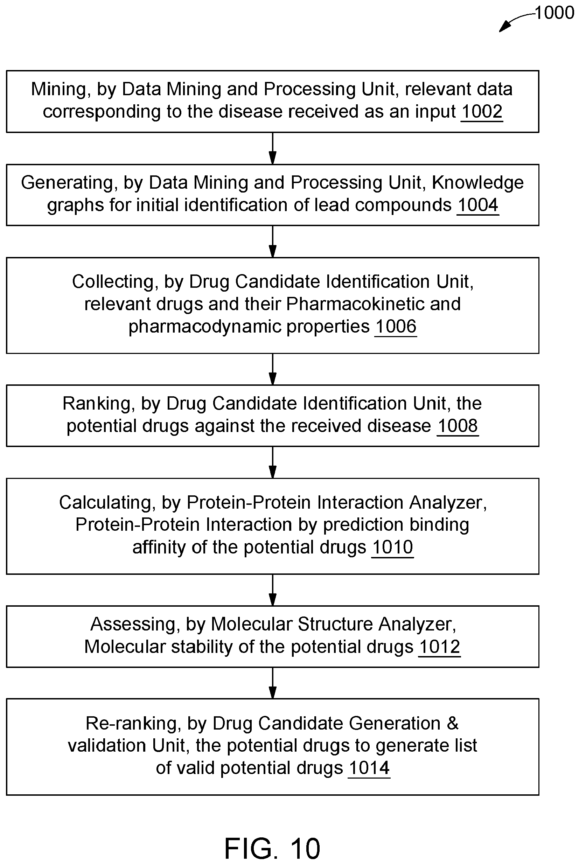

Referring now to FIG. 10, a detailed exemplary method 1000 for selecting candidate drug compounds for a disorder through AI-based drug repurposing is depicted via a flow chart, in accordance with an embodiment of the present disclosure. In an embodiment, the method 1000 may be implemented by the drug candidate identification device 102. The method 1000 includes mining, by the drug mining and processing unit 204, relevant data corresponding to the disease received as an input, at step 1002. The drug mining and processing unit 204 identifies disease data 220 corresponding to the disease received as an input. The drug mining and processing unit 204 implements an NLP algorithm to explore larger resources to gather valid and relevant information about the disease/disorder from databases such as, but not limited to, PubMed, DrugBank, PharmGKB, etc.

Further, the method 1000 includes generating, by the drug mining and processing unit 204, knowledge graphs for initial identification of lead compounds, at step 1004. The drug mining and processing unit 204 generates an extensive semantic knowledge graph from the identified drug-related properties (e.g., drug-gene, gene-gene, and gene-phenotype relationships) to identify valid enzymes and proteins.

The drug mining and processing unit 204 identifies a list of drugs, diseases, and proteins using a custom trained BERT model built from Bio-BERT embedding as a Named Entity Recognizer (NER). After NER model, the drug candidate identifying unit 206 constructs more complete lexicons of drugs, genes, and phenotypes using distributional semantics (pharmacogenomic relationships). Further, the drug mining and processing unit 204 identifies drug-gene, gene-gene, and gene-phenotype relationships using the curated lexicons.

Further, the method 1000 includes collecting, by the drug candidate identifying unit 206, relevant drugs and their pharmacokinetic and pharmacodynamic properties, at step 1006. The drug candidate identifying unit 206 identifies a set of lead compounds and associated pharmacokinetic and pharmacodynamic properties through the clinical information processing unit 214.

The drug candidate identifying unit 206 ranks each of the set of lead compounds to identify relevant and appropriate drugs for treating the target disorder using the associated pharmacokinetic and pharmacodynamic properties as features. Further, the drug candidate identifying unit 206 creates GMMs based on the features to classify each of the set of lead compounds into one or more clusters.

Further, the method 1000 includes ranking, by the drug candidate identifying unit 206, the potential drugs against the received disease, at step 1008. The drug candidate identifying unit 206 ranks each of the set of lead compounds to identify relevant and appropriate drugs for treating the target disorder.

The drug candidate identifying unit 206 assigns a custom score to each of the one or more clusters based on available historical clinical feature information. Further, the drug candidate identifying unit 206 validates each of the set of lead compounds based on historical information of other existing diseases. In some embodiments, the drug candidate identifying unit 206 assigns a rank corresponding to each of the one or more clusters.

Upon assigning the custom score, the drug candidate identifying unit 206 assigns an initial rank to each of lead compounds corresponding to the target disorder within a cluster using a combination of deep learning-based ranking algorithms (such as, RankNet and LambdaMart).

Further, the method 1000 includes calculating, by the protein-protein interaction analyzer 210, protein-protein interaction by prediction binding affinity of the potential drugs, at step 1010. The protein-protein interaction analyzer 210 estimates protein-protein interaction by predicting binding affinity score of each of the set of lead compounds. The protein-protein interaction analyzer 210 predicts the binding affinity score of a lead compound corresponding to the amino acid sequence of the target protein-protein interaction complex.

Further, the protein-protein interaction analyzer 210 generates drug and protein embeddings through AI-based encoder networks (such as, the drug encoder 604 and the target encoder 606), and then concatenates the drug and protein embeddings into a decoder network (such as, the decoder 608) for the prediction of binding affinity score.

Further, the method 1000 includes assessing, by molecular structure analyzer 212, molecular stability of the potential drugs, at step 1012. The molecular structure analyzer 212 assesses each of the set of lead compounds in terms of molecular stability corresponding to the target protein-protein interaction complex. Further, the molecular structure analyzer 212 generates a novel compound using deep learning algorithms. It may be noted that the novel compound is an ideal binding molecule to the target protein-protein interaction complex. Further, the molecular structure analyzer 212 assesses molecular stability of the novel compound by comparing the novel compound with existing drug compounds.

The molecular structure analyzer 212 collects common physiochemical features and applies PCA on the physiochemical features to determine whether the novel compound is transformed accordingly. Further, the molecular structure analyzer 212 calculates a molecular structure stability score for each of the set of lead compounds in comparison with molecular structure of the novel compound.

Further, the method 1000 includes re-ranking, by drug candidate generation and validation unit 208, the potential drugs to generate list of valid potential drugs, at step 1014. The drug candidate generation and validation unit 208 re-ranks each of the set of lead compounds based on the identified binding affinity score and the molecular structure stability score and intermediate clinical trial data corresponding to each of the set of lead compounds. The drug candidate generation and validation unit 208 shares the re-ranked set of lead compounds with the corresponding intermediate clinical trial data is shared as an output.

The output includes the set of lead compounds including drug candidate compounds, identified corresponding to the target disorder. The set of lead compounds may be validated in further clinical trials. For example, the system 200 may correctly identify drugs shortlisted by WHO for solidarity trials for COVID-19. Additionally, the system 200 may identify drugs that may not make through the clinical trials by assigning a lower rank to such drugs.

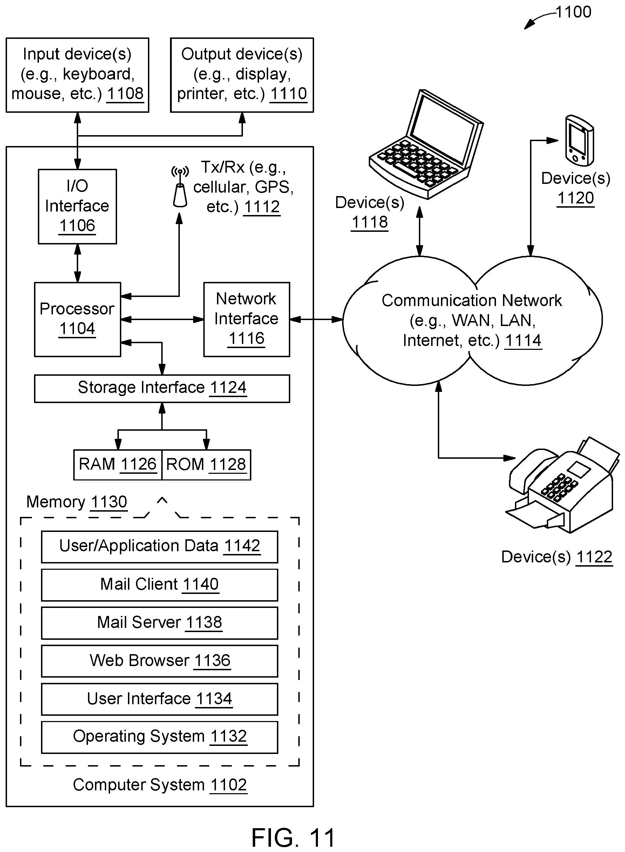

The disclosed methods and systems may be implemented on a conventional or a general-purpose computer system, such as a personal computer (PC) or server computer. Referring now to FIG. 11, a block diagram of an exemplary computer system 1102 for implementing embodiments consistent with the present disclosure is illustrated. Variations of computer system 1102 may be used for implementing the system 100 for selecting candidate drug compounds for a disorder through AI-based drug repurposing. Computer system 1102 may include a central processing unit ("CPU" or "processor") 1104. Processor 1104 may include at least one data processor for executing program components for executing user-generated or system-generated requests. A user may include a person, a person using a device such as such as those included in this disclosure, or such a device itself. The processor may include specialized processing units such as integrated system (bus) controllers, memory management control units, floating point units, graphics processing units, digital signal processing units, etc. The processor may include a microprocessor, such as AMD^{®} ATHLON^{®}, DURON^{®} OR OPTERON^{®}, ARM's application, embedded or secure processors, IBM^{®} POWERPC^{®}, INTEL^{®} CORE^{®} processor, ITANIUM^{®} processor, XEON^{®} processor, CELERON^{®} processor or other line of processors, etc. The processor 1104 may be implemented using mainframe, distributed processor, multi-core, parallel, grid, or other architectures. Some embodiments may utilize embedded technologies like application-specific integrated circuits (ASICs), digital signal processors (DSPs), Field Programmable Gate Arrays (FPGAs), etc.

Processor 1104 may be disposed in communication with one or more input/output (I/O) devices via I/O interface 1106. The I/O interface 1106 may employ communication protocols/methods such as, without limitation, audio, analog, digital, monoaural, RCA, stereo, IEEE-1394, near field communication (NFC), FireWire, Camera Link^{®}, GigE, serial bus, universal serial bus (USB), infrared, PS/2, BNC, coaxial, component, composite, digital visual interface (DVI), high-definition multimedia interface (HDMI), radio frequency (RF) antennas, S-Video, video graphics array (VGA), IEEE 802.n /b/g/n/x, Bluetooth, cellular (e.g., code-division multiple access (CDMA), high-speed packet access (HSPA+), global system for mobile communications (GSM), long-term evolution (LTE), WiMAX, or the like), etc.

Using the I/O interface 1106, the computer system 1102 may communicate with one or more I/O devices. For example, the input device 1108 may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, altimeter, gyroscope, proximity sensor, or the like), stylus, scanner, storage device, transceiver, video device/source, visors, etc. Output device 1110 may be a printer, fax machine, video display (e.g., cathode ray tube (CRT), liquid crystal display (LCD), light-emitting diode (LED), plasma, or the like), audio speaker, etc. In some embodiments, a transceiver 1112 may be disposed in connection with the processor 1104. The transceiver may facilitate various types of wireless transmission or reception. For example, the transceiver may include an antenna operatively connected to a transceiver chip (e.g., TEXAS INSTRUMENTS^{®} WILINK WL1286^{®}, BROADCOM^{®} BCM4550IUB8^{®}, INFINEON TECHNOLOGIES^{®} X-GOLD 1436-PMB9800^{®} transceiver, or the like), providing IEEE 802.11a/b/g/n, Bluetooth, FM, global positioning system (GPS), 2G/3G HSDPA/HSUPA communications, etc.

In some embodiments, the processor 1104 may be disposed in communication with a communication network 1116 via a network interface 1114. The network interface 1114 may communicate with the communication network 1116. The network interface may employ connection protocols including, without limitation, direct connect, Ethernet (e.g., twisted pair 10/100/1000 Base T), transmission control protocol/internet protocol (TCP/IP), token ring, IEEE 802.11a/b/g/n/x, etc. The communication network 1116 may include, without limitation, a direct interconnection, local area network (LAN), wide area network (WAN), wireless network (e.g., using Wireless Application Protocol), the Internet, etc. Using the network interface 1114 and the communication network 1116, the computer system 1102 may communicate with devices 1118, 1120, and 1122. These devices may include, without limitation, personal computer(s), server(s), fax machines, printers, scanners, various mobile devices such as cellular telephones, smartphones (e.g., APPLE^{®} IPHONE^{®}, BLACKBERRY^{®} smartphone, ANDROID^{®} based phones, etc.), tablet computers, eBook readers (AMAZON^{®} KINDLE^{®}, NOOK^{®} etc.), laptop computers, notebooks, gaming consoles (MICROSOFT^{®} XBOX^{®}, NINTENDO^{®} DS^{®}, SONY^{®} PLAYSTATION^{®}, etc.), or the like. In some embodiments, the computer system 1102 may itself embody one or more of these devices.

In some embodiments, the processor 1104 may be disposed in communication with one or more memory devices 1130 (e.g., RAM 1126, ROM 1128, etc.) via a storage interface 1124. The storage interface may connect to memory devices 1130 including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), STD Bus, RS-232, RS-422, RS-485, I2C, SPI, Microwire, 1-Wire, IEEE 1284, Intel^{®} QuickPathInterconnect, InfiniBand, PCIe, etc. The memory drives may further include a drum, magnetic disc drive, magneto-optical drive, optical drive, redundant array of independent discs (RAID), solid-state memory devices, solid-state drives, etc.

The memory devices 1130 may store a collection of program or database components, including, without limitation, an operating system 1132, user interface application 1134, web browser 1136, mail server 1138, mail client 1140, user/application data 1142 (e.g., any data variables or data records discussed in this disclosure), etc. The operating system 1132 may facilitate resource management and operation of the computer system 1102. Examples of operating systems include, without limitation, APPLE^{®} MACINTOSH^{®} OS X, UNIX, Unix-like system distributions (e.g., Berkeley Software Distribution (BSD), FreeBSD, NetBSD, OpenBSD, etc.), Linux distributions (e.g., RED HAT^{®}, UBUNTU^{®}, KUBUNTU^{®}, etc.), IBM^{®} OS/2, MICROSOFT^{®} WINDOWS^{®} (XP^{®}, Vista^{®}/7/8, etc.), APPLE^{®} IOS^{®}, GOOGLE^{®} ANDROID^{®}, BLACKBERRY^{®} OS, or the like. User interface 1134 may facilitate display, execution, interaction, manipulation, or operation of program components through textual or graphical facilities. For example, user interfaces may provide computer interaction interface elements on a display system operatively connected to the computer system 1102, such as cursors, icons, check boxes, menus, scrollers, windows, widgets, etc. Graphical user interfaces (GUIs) may be employed, including, without limitation, APPLE^{®} MACINTOSH^{®} operating systems' AQUA^{®} platform, IBM^{®} OS/2^{®}, MICROSOFT^{®} WINDOWS^{®} (e.g., AERO^{®}, METRO^{®}, etc.), UNIX X-WINDOWS, web interface libraries (e.g., ACTIVEX^{®}, JAVA^{®}, JAVASCRIPT^{®}, AJAX^{®}, HTML, ADOBE^{®} FLASH^{®}, etc.), or the like.

In some embodiments, the computer system 1102 may implement a web browser 1136 stored program component. The web browser may be a hypertext viewing application, such as MICROSOFT^{®} INTERNET EXPLORER^{®}, GOOGLE^{®} CHROME^{®}, MOZILLA^{®} FIREFOX^{®}, APPLE^{®} SAFARI^{®}, etc. Secure web browsing may be provided using HTTPS (secure hypertext transport protocol), secure sockets layer (SSL), Transport Layer Security (TLS), etc. Web browsers may utilize facilities such as AJAX^{®}, DHTML, ADOBE^{®} FLASH^{®}, JAVASCRIPT^{®}, JAVA^{®}, application programming interfaces (APIs), etc. In some embodiments, the computer system 1102 may implement a mail server 1138 stored program component. The mail server may be an Internet mail server such as MICROSOFT^{®} EXCHANGE^{®}, or the like. The mail server may utilize facilities such as ASP, ActiveX, ANSI C++/C#, MICROSOFT .NET^{®} CGI scripts, JAVA^{®}, JAVASCRIPT^{®}, PERL^{®}, PHP^{®}, PYTHON^{®}, WebObjects, etc. The mail server may utilize communication protocols such as internet message access protocol (IMAP), messaging application programming interface (MAPI), MICROSOFT^{®} EXCHANGE^{®}, post office protocol (POP), simple mail transfer protocol (SMTP), or the like. In some embodiments, the computer system 1102 may implement a mail client 1140 stored program component. The mail client may be a mail viewing application, such as APPLE MAIL^{®}, MICROSOFT ENTOURAGE^{®}, MICROSOFT OUTLOOK^{®}, MOZILLA THUNDERBIRD^{®}, etc.

In some embodiments, computer system 1102 may store user/application data 1142, such as the data, variables, records, etc. (e.g., the set of predictive models, the plurality of clusters, set of parameters (batch size, number of epochs, learning rate, momentum, etc.), accuracy scores, competitiveness scores, ranks, associated categories, rewards, threshold scores, threshold time, and so forth) as described in this disclosure. Such databases may be implemented as fault-tolerant, relational, scalable, secure databases such as ORACLE^{®} OR SYBASE^{®}. Alternatively, such databases may be implemented using standardized data structures, such as an array, hash, linked list, struct, structured text file (e.g., XML), table, or as object-oriented databases (e.g., using OBJECTSTORE^{®}, POET^{®}, ZOPE^{®}, etc.). Such databases may be consolidated or distributed, sometimes among the various computer systems discussed above in this disclosure. It is to be understood that the structure and operation of the any computer or database component may be combined, consolidated, or distributed in any working combination.

Thus, the disclosed method and system try to overcome the technical problem of selecting candidate drug compounds for a disorder through AI-based drug repurposing. The method and system significantly reduce duration of drug discovery processes. Especially in pandemic or epidemic like situation (for example, COVID-19 pandemic), wherein it takes more than years to discover a drug to treat the disorder, discovering repurposed drugs is shorter as safety and toxicology studies are already done. Further, the method and system significantly reduce cost of licensing and marketing. Cost of bringing a repurposed drug into market is very less compared to a new drug discovery, especially with AI-based computational methods. Further, the method and system minimize risk of failure of drugs against target molecules. AI limits scope by shortlisting potential drug candidates. The proposed method enables shortlisting high ranked drugs, which can used to cure a disease. Further, the method and system provide a potential to improve and assist drug discovery process and planning, being an evidence-based and data driven medicinal solution. Further, the method and system provide safety as toxicity and other properties of the drugs are pre-determined.

As will be appreciated by those skilled in the art, the techniques described in the various embodiments discussed above are not routine, or conventional, or well understood in the art. The techniques discussed above provide for selecting candidate drug compounds for a disorder through AI-based drug repurposing. The techniques implement transformer network to generate semantic knowledge graphs for the initial identification of lead compounds. The techniques further incorporate clinical features along with available intermediate clinical trial information into the model. The techniques further predict drug target interactions using encoder, decoder and transformer network by predicting the free binding energy (binding affinity). The techniques further generate a drug sequence using attention model with an AI-based encoder-decoder network and validate the generated sequence for the desired drug properties. The techniques further provide for similarity matching of the generated sequence with the shortlisted drug candidates and providing the validated potential drug candidates.

In light of the above mentioned advantages and the technical advancements provided by the disclosed method and system, the claimed steps as discussed above are not routine, conventional, or well understood in the art, as the claimed steps enable the following solutions to the existing problems in conventional technologies. Further, the claimed steps clearly bring an improvement in the functioning of the device itself as the claimed steps provide a technical solution to a technical problem.

The specification has described method and system for selecting candidate drug compounds for a disorder through AI-based drug repurposing. The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A method for selecting candidate drug compounds for a disorder through Artificial Intelligence-based, AI-based, drug repurposing, the method comprising:
extracting, from a plurality of databases through a Natural Language Processing, NLP, algorithm, by a drug candidate identification device, data related to the disorder, wherein the extracted data identifies a target protein-protein interaction complex associated with the disorder;
generating, by the drug candidate identification device, a semantic knowledge graph for the disorder based on the extracted data to identify a set of lead compounds corresponding to the target protein-protein interaction complex;
assigning, by the drug candidate identification device, an initial rank to each of the set of lead compounds based on historical clinical information of each of the set of lead compounds and the semantic knowledge graph, through a predictive model, wherein the predictive model comprises at least one of a clustering algorithm and a probabilistic algorithm;
calculating, by the drug candidate identification device, a binding affinity score corresponding to each of the set of lead compounds and the target protein-protein interaction complex through an AI-based encoder-decoder model;
for each of the set of lead compounds, determining, by the drug candidate identification device, a molecular structure stability score based on interaction of a molecular structure of a lead compound with a molecular structure of the target protein-protein interaction complex through a deep learning model; and
assigning, by the drug candidate identification device, a final rank to each of the set of lead compounds based on the binding affinity score, the molecular structure stability score, and intermediate clinical trial data corresponding to each of the set of lead compounds.

2. The method of claim 1, wherein generating the semantic knowledge graph to identify the set of lead compounds comprises:
determining one or more target proteins from the target protein-protein interaction complex;
validating the one or more target proteins based on manually curated databases; and
upon successfully validating, identifying the set of lead compounds corresponding to each of the one or more target proteins based on drug repositories.

3. The method of claim 1 or 2, wherein assigning the initial rank to each of the set of lead compounds comprises:
extracting pharmacokinetic and pharmacodynamic properties corresponding each of the set of lead compounds from the semantic knowledge graph;
classifying each of the set of lead compounds into one or more clusters based on the pharmacokinetic and pharmacodynamic properties through a clustering algorithm;
assigning a custom score to each of the one or more clusters based on the historical clinical information of each of the set of lead compounds; and
assigning the initial rank to each of the set of lead compounds in each of the one or more clusters through a probabilistic model.

4. The method of any of claims 1 to 3, wherein calculating the binding affinity score through an AI-based encoder-decoder model comprises:
generating a drug embedding for each of the set of lead compounds through a drug encoder model;
generating a target embedding for the target protein-protein interaction complex through a target encoder model; and
determining the binding affinity score corresponding to a combination of the drug embedding and the target embedding through a decoder model.

5. The method of any of claims 1 to 4, wherein determining a molecular structure stability score of each of the set of lead compounds through a deep learning model comprises:
generating a novel compound corresponding to a binding site of the target protein-protein interaction complex through the deep learning model, wherein the binding affinity score of the novel compound with the target protein-protein interaction complex is above a predefined threshold;
determining a molecular structure of the novel compound through the deep learning model;
validating a set of crystallographic properties associated with the molecular structure of the novel compound; and
upon successfully validating, comparing the molecular structure of the novel compound with molecular structure of each of the set of lead compounds.

6. The method of claim 5, wherein comparing the molecular structure of the novel compound with molecular structure of each of the set of lead compounds comprises:
estimating similarities between the molecular structure of the novel compound and the molecular structure of each of the set of lead compounds through a drug encoder model; and
assigning cosine similarity scores to each of the set of lead compounds based on the estimated drug similarities.

7. The method of any of claims 1 to 6, further comprising receiving intermediate clinical trial data corresponding to each of the set of lead compounds from an intermediate clinical trial repository.

8. A system for selecting candidate drug compounds for a disorder through Artificial Intelligence -based, AI-based, drug repurposing, the system comprising:
a processor; and
a memory communicatively coupled to the processor, wherein the memory stores processor instructions which, when executed by the processor, cause the processor to:
extract, from a plurality of databases through a Natural Language Processing, NLP, algorithm, data related to the disorder, wherein the extracted data identifies a target protein-protein interaction complex associated with the disorder;
generate a semantic knowledge graph for the disorder based on the extracted data to identify a set of lead compounds corresponding to the target protein-protein interaction complex;
assign an initial rank to each of the set of lead compounds based on historical clinical information of each of the set of lead compounds and the semantic knowledge graph, through a predictive model, wherein the predictive model comprises at least one of a clustering algorithm and a probabilistic algorithm;
calculate a binding affinity score corresponding to each of the set of lead compounds and the target protein-protein interaction complex through an AI-based encoder-decoder model;
for each of the set of lead compounds, determine a molecular structure stability score based on interaction of a molecular structure of a lead compound with a molecular structure of the target protein-protein interaction complex through a deep learning model; and
assign a final rank to each of the set of lead compounds based on the binding affinity score, the molecular structure stability score, and intermediate clinical trial data corresponding to each of the set of lead compounds.

9. The system of claim 8, wherein to generate the semantic knowledge graph to identify the set of lead compounds, the processor instructions, when executed by the processor, cause the processor to:
determine one or more target proteins from the target protein-protein interaction complex;
validate the one or more target proteins based on manually curated databases; and
upon successfully validating, identify the set of lead compounds corresponding to each of the one or more target proteins based on drug repositories.

10. The system of claim 8 or claim 9, wherein to assign the initial rank to each of the set of lead compounds, the processor instructions, when executed by the processor, cause the processor to:
extract pharmacokinetic and pharmacodynamic properties corresponding each of the set of lead compounds from the semantic knowledge graph;
classify each of the set of lead compounds into one or more clusters based on the pharmacokinetic and pharmacodynamic properties through a clustering algorithm;
assign a custom score to each of the one or more clusters based on the historical clinical information of each of the set of lead compounds; and
assign the initial rank to each of the set of lead compounds in each of the one or more clusters through a probabilistic model.

11. The system of any of claims 8 to 10, wherein to calculate the binding affinity score through an AI-based encoder-decoder model, the processor instructions, when executed by the processor, cause the processor to:
generate a drug embedding for each of the set of lead compounds through a drug encoder model;
generate a target embedding for the target protein-protein interaction complex through a target encoder model; and
determine the binding affinity score corresponding to a combination of the drug embedding and the target embedding through a decoder model.

12. The system of any of claims 8 to 11, wherein to determine a molecular structure stability score of each of the set of lead compounds through a deep learning model, the processor instructions, when executed by the processor, cause the processor to:
generate a novel compound corresponding to a binding site of the target protein-protein interaction complex through the deep learning model, wherein the binding affinity score of the novel compound with the target protein-protein interaction complex is above a predefined threshold;
determine a molecular structure of the novel compound through the deep learning model;
validate a set of crystallographic properties associated with the molecular structure of the novel compound; and
upon successfully validating, compare the molecular structure of the novel compound with molecular structure of each of the set of lead compounds.

13. The system of claim 12, wherein to compare the molecular structure of the novel compound with molecular structure of each of the set of lead compounds, the processor instructions, when executed by the processor, cause the processor to:
estimate similarities between the molecular structure of the novel compound and the molecular structure of each of the set of lead compounds through a drug encoder model; and
assign cosine similarity scores to each of the set of lead compounds based on the estimated drug similarities.

14. The system of any of claims 8 to 13, wherein the processor instructions, when executed by the processor, cause the processor to receive intermediate clinical trial data corresponding to each of the set of lead compounds from an intermediate clinical trial repository.

15. A computer-readable medium storing computer-executable instructions for selecting candidate drug compounds for a disorder through Artificial Intelligence AI-based drug repurposing, the computer-executable instructions configured for causing at least one processor to execute the method of any of claims 1 to 7.
